Europäisches Patentamt

European Patent Office

Office européen des brevets

(19)

(11) **EP 1 112 775 B1**

(12) **EUROPEAN PATENT SPECIFICATION**

(45) Date of publication and mention
of the grant of the patent:
**29.06.2005 Bulletin 2005/26**

(51) Int Cl.$^7$: **B01J 23/62**, C07C 67/055

(21) Application number: **00301200.2**

(22) Date of filing: **16.02.2000**

(54) **Catalyst for the preparation of allyl acetate**

Katalysator zur Herstellung von Allylacetat

Catalyseur pour la fabrication d'acétate d'allyle

(84) Designated Contracting States:
**AT BE CH CY DE DK ES FI FR GB GR IE IT LI LU
MC NL PT SE**

(30) Priority: **30.12.1999 GB 9930830**

(43) Date of publication of application:
**04.07.2001 Bulletin 2001/27**

(73) Proprietor: **Dairen Chemical Corporation
Taipei (TW)**

(72) Inventors:
• **Chen, Shien-Change
Taipei (TW)**
• **Hsu, Liang-An
Kaohsiung (TW)**
• **Lin, Fu-Shen
Kaohsiung (TW)**
• **Jang, Pi-Fwu
Kaohsiung (TW)**

(74) Representative: **Ablett, Graham Keith et al
Ablett & Stebbing,
Caparo House,
101-103 Baker Street
London W1M 1FD (GB)**

(56) References cited:
**EP-A- 0 519 436        DE-B- 1 261 847
GB-A- 1 177 515        US-A- 3 925 452
US-A- 3 957 688        US-A- 4 008 180
US-A- 4 717 421**

Remarks:
The file contains technical information submitted
after the application was filed and not included in this
specification

**Description**

## FIELD OF THE INVENTION

This invention relates to a catalyst which is comprised of palladium metal as the main catalyst, a mixture of tin and additional metal(s) as the promoter, in combination with an alkali or alkaline earth metal compound, supported on the outer surface of a porous carrier. The catalyst is used in the process for producing allyl acetate through the oxacylation of propylene, acetic acid and oxygen in a vapor phase.

## BACKGROUND OF THE INVENTION

Previously, in the production of allyl acetate through the oxacylation of propylene, acetic acid and oxygen in a vapor phase, a silica carrier impregnated with palladium only was the main catalyst, and alkali or alkaline earth metal compound was used as the activator (USP 3925452). In order to get better activity from this catalyst, the oxacylation should be performed at higher temperatures. Under these conditions, the formation of carbon dioxide byproduct was increased, and the space time yield (STY, the yield per hour per liter of the catalyst) of allyl acetate was unable to be promoted even by increasing the amounts of palladium or the activator. Generally, by only using palladium as the catalyst during the oxacylation process, the space time yield of allyl acetate would not exceed 60 (g/hr/l of catalyst), and the selectivity of such a catalyst to allyl acetate would only reach 87%. In other words, most of propylene reactant was burned into carbon dioxide or converted into other byproducts. Apparently, when only palladium and the activator were used as the catalyst, the catalytic ability of the catalyst was rather low, and waste resulted from the complete burning of propylene into carbon dioxide, which adversely influenced the industrial process.

In order to improve on this drawback, in the preparation process of the catalyst for oxacylation reaction, other metals were added so as to increase the activity and selectivity of the catalyst (USP 3917676). Therefore, most of the compositions of the catalysts were comprised of, not only palladium, the main catalyst, and alkali or alkaline earth metal, the activator, but also other metals, as the promoters. For example, along with catalysts with the combination of the main catalyst palladium and the promoters of potassium, bismuth and barium (USP 4571431), the combination of the main catalyst palladium and the promoters of copper, lead, ruthenium and rhenium (EP 0361484), etc. have been disclosed. Among these, a catalyst with the combination of the main catalyst palladium and the promoter copper exhibited higher activity and selectivity (USP 5011980).

For the purpose of high catalytic activity and high catalyst selectivity, besides the addition of other metals as the promoter in preparing the catalyst, a certain amount of water as a diluent should be added into the feeding materials of propylene, acetic acid and oxygen for performing the oxacylation reaction, in the traditional process of producing ally acetate. If moisture content was under a certain ratio, the catalytic activity and life of the catalyst could not be retained and would deteriorate rapidly. Usually, the addition of the water diluent would limit the yield of the final product. Further, the final product, allyl acetate, should be purified after the oxacylation, which would result in consuming and wasting energy of the whole process, and the economical effect could not be attained.

In the presence of the catalyst produced in this invention, while no water is added into the reactant materials for the oxacylation process, or only a small amount of water is added in accordance with the requirement of the process, not only will the catalytic activity and life of the catalyst be retained and not deteriorate, but a high catalytic activity and high selectivity will be attained. Therefore, the energy consumed and wasted resulting from the addition of water can be avoided, and the economical effect of the oxacylation process can be greatly increased.

## SUMMARY OF THE INVENTION

This invention relates to a catalyst according to claim 1. The catalyst is used in the process for producing allyl acetate through the oxacylation of propylene, acetic acid and oxygen in a vapor phase. The catalyst of the present invention exhibits high catalytic activity, high catalytic selectivity and high catalytic life, which greatly increases the economic utility of the oxacylation process.

## DETAILED DESCRIPTION OF THE INVENTION

The porous carriers which are suitable for preparing the catalyst for oxacylation of the present invention are alumina, silica gel, silica, active carbon, silicon carbide, diatomaceous earth, pumice and the like, while among these, silica and alumina are preferable.

The main catalyst metal of the catalyst for oxacylaion of the present invention is palladium; the metal content thereof, based on the weight of the carrier, is 0.1 to 5.0 weight %, preferably 0.3 to 1.5 weight %. The promoter metal of the catalyst for oxacylation of the present invention is a mixture of tin and additional metal(s) selected from the group consisting of gold, copper, cadmium, bismuth, cerium and a mixture thereof, while among these, a mixture of tin and gold is preferable; the metal content thereof, based on the weight of the carrier, is 0.01 to 5.0 weight %, preferably 0.02 to 1.0 weight %. The activator of the catalyst for oxacylation of the present invention is an alkali or alkaline earth metal compound, the examples thereof being the hydroxides, acetates, nitrates and bicarbonates of potassium, sodium, cesium, magnesium, barium and the like, while among these, potassium salts are preferable, and potassium acetate is even more preferable. The content thereof, based on the weight of the carrier, is 4 to 10 weight %.

Traditionally, the preparation method of the catalyst for oxacylation was essentially comprised of the following steps: (1) a carrier was impregnated with an aqueous solution of soluble palladium ions and metal ions of the promoter; (2) the impregnated carrier was immersed in an alkali solution, so that the soluble palladium ions and metal ions of the promoter were precipitated on the surface layer of the carrier and formed into insoluble oxidative state palladium and promoter metal; (3) the treated carrier was washed with water to remove soluble ions produced during the precipitation; (4) the oxidative state palladium and promoter metal supported on the treated carrier were then reduced to the metallic state; (5) the reduced carrier in (4) was impregnated with a solution of an alkali or alkaline earth metal compound; and (6) the impregnated carrier in (5) was dried. The term "oxidative state" used herein according to the present invention means a metal in a cationic state, for example, oxidative state palladium means $Pd^{2+}$.

The catalyst for oxacylation of the present invention is prepared mainly in accordance with the traditional method. After the oxidative state palladium and promoter metal are supported on the surface of the porous carrier, this not-reduced yet catalyst is placed in a reactor and the reducing step is performed under suitable reductive conditions using gaseous or liquid reducing agents. The examples of the reducing agents are amines, carbon monoxide, hydrogen, alkene, aldehydes and hydrazines. When gaseous reducing agents are used, it is preferable to dilute the gaseous reducing agent with inert gas (such as nitrogen gas). The amount of the reducing agent used depends on the amounts of the palladium and the promoter metal, the equivalents used thereof usually being at least 1 to 1.5 times of the equivalents required to reduce the catalyst. If necessary, more reducing agent can be used. After the reducing process, the reduced catalyst is washed with deionized water until the chloride ions are completely removed and then dried. After drying, the reduced catalyst is impregnated with an aqueous solution containing an alkali or alkaline earth metal compound. Finally, the catalyst is dried at a temperature between 80 to 150°C.

A certain amount of the above prepared catalyst for oxacylation is placed in a reacting tube with an inner diameter of 20mm and a length of 2.0 m. Under a specific pressure at the inlet of the reacting tube, the reactant feeding gases are introduced into the tube at a reacting temperature set according to the activity of the catalyst. These reactant feedings comprise propylene, nitrogen, acetic acid, oxygen and water, wherein the content of propylene is 20 to 50 volume %; the content of nitrogen is 20 to 60 volume %; the content of acetic acid is 5 to 25 volume %; the content of oxygen is 5 to 10 volume %; and the content of water is 0 to 15 volume %, preferably 0 to 10 volume %. The catalyst for oxacylation of the present invention is characterized in that, the catalytic activity and life of the catalyst can be retained and will not deteriorate while no water is added into the reactant composition, or if only a small amount of water is added in the oxacylation process.

The operation temperature of the above oxacylation process is in the range of 100°C to 250°C, preferably 140°C to 200°C; the operation pressure is in the range of 0 to 15 kg/cm$^2$•g, preferable 5 to 10 kg/cm$^2$•g.

The yield of allyl acetate is determined by analyzing the composition at the exit when the oxacylation process is carried out for a definite time.

Generally, the selection of a catalyst in the industry is based on the catalytic activity (STY). The catalytic activity can be calculated basically according to the following formula:

The activity of a catalyst:

$$STY(\text{space time yield}) = \frac{\text{weight of allyl acetates produced(g)}}{\text{volume(l) of catalyst x sampling time(hr)}}$$

The selectivity of a catalyst:

$$\text{Allyl acetate selectivity} = \frac{\text{moles of allyl acetate produced}}{\text{moles of allyl acetate produced} + 1/3 \text{ moles of CO}_2 \text{ produced}}$$

It is confirmed from the evaluation of the catalytic activity in the oxacylation process practical application that the catalyst for oxacylation in accordance with the present invention not only provides higher activity of the whole oxacyltion reaction of propylene, acetic acid and oxygen, but also prolongs its own life. That is, compared to the convenfional catalysts, the catalyst of the present invention is able to yield more allyl acetate per unit volume of catalyst in the reactor and per unit time, while no water or only a small amount of water is added in the oxacylation process, and the conditions of the oxacylation reaction (such as pressure, temperature, oxygen concentration) remain constant. Thus, the energy wasted resulting from the traditional process by using large amounts of water can be reduced. Moreover, if the productive yield remains constant, not only can the reacting temperature be decreased, but also the selectivity of the reaction can be higher, which leads to less production of carbon dioxide and less product loss during the removal of carbon dioxide. Thus, the unit raw material consumption will be lower. This is beneficial to the industrial production of allyl acetate.

The present invention will be further described with reference to the following Examples and Comparative Examples.

Example 1

The carrier employed in this Example was a porous carrier of alumina/silica with an outer diameter of 5 mm and available from SUD-CHEMIE AG. This carrier had a surface area of 100 to 120 $m^2/g$, a pore volume of 0.7 to 0.9 ml/g and a bulk density of 600 g/l. The metal component-supporting catalyst was prepared according to the following steps:

Step 1): An aqueous $Na_2PdCl_4$ solution with weight of 2.2 kg containing 15 weight % of palladium was added into a mixture of an aqueous $SnCl_2$ solution with weight of 0.5 kg containing 15 weight % of tin and an aqueous $HAuCl_4$ solution with weight of 0.5 kg containing 30 weight % of gold. The mixture was then diluted with deionized water till total volume was 37.2 liters. One-hundred liters of alumina/silica carrier was placed in an impregnating tank with rotation rate of 24 turns per minute. The mixture was added into the tank rapidly.

Step 2): Hot air was passed through to dry the carrier. The temperature of the hot air was lower than 120°C.

Step 3): Twenty-eight weight % of NaOH solution (about 60 kg) was added to the dried catalyst. The originally soluble chloride state palladium, tin and gold were transformed into insoluble hydroxide state palladium, tin and gold.

Step 4): The impregnated catalyst carrier after drying was placed in a reducing reactor. The reducing gases were passed into the reactor, wherein the reducing gases could be diluted with other inert gases. The hydroxide state metal catalyst was reduced into a metallic state catalyst.

Step 5): The above catalyst was washed to remove chloride ions until the catalyst was free of chloride ions.

Step 6): The catalyst carrier was dried as in step 2).

Step 7): An adequate amount of potassium acetate was added into the dried catalyst carrier, so that each liter of the catalyst contained 30 g weight of potassium acetate.

Step 8): The catalyst carrier was dried as in step 2).

After the above steps, a catalyst containing 3.3 g/l of palladium, 0.75 g/l of tin, 1.5 g/l of gold and 30 g/l of potassium acetate was obtained, wherein all palladium, tin and gold were well distributed on the surface of the carrier.

Four hundred and fifty milliliters of the catalyst thus obtained was charged into a reacting tube with an inner diameter of 20 mm and a length of 2.0 m. Under a pressure of 7 $kg/cm^2$ (gauge pressure) at the inlet of the reactor, the reacting gaseous mixture was introduced into the reactor at a temperature of 140°C. The gaseous mixture was comprised of 41 volume % of propylene, 43 volume % of nitrogen gas, 10 volume % of acetic acid and 6 volume % of oxygen. When the composition at the exit was analyzed in a definite time, the activity and the selectivity of the catalyst were calculated. The results are listed in Table 1.

When the activity and the selectivity of the catalyst were evaluated, the crude product at the exit of the reactor was cooled with chilled water, and the composition was analyzed by Shimadzu Gas Chromatography. The flow rate of the gases was determined by Shinagawa Dry Gas Meter.

Example 2

The catalyst was prepared by the same method as in Example 1 except that, in step 1), an aqueous $Na_2PdCl_4$ solution with weight of 2.2 kg containing 15 weight % of palladium, an aqueous $SnCl_2$ solution with weight of 0.5 kg containing 15 weight % of tin and an aqueous $CuCl_2$ solution with weight of 0.5 kg containing 15 weight % of copper were prepared.

This catalyst was evaluated by the same method as in Example 1, and the results are listed in Table 1.

Example 3

The catalyst was prepared exactly by the same method as in Example 1, i.e., an aqueous $Na_2PdCl_4$ solution with weight of 2.2 kg containing 15 weight % of palladium, an aqueous $SnCl_2$ solution with weight of 0.5 kg containing 15 weight % of tin and an aqueous $HAuCl_4$ solution with weight of 0.5 kg containing 30 weight % of gold were prepared.

This catalyst was evaluated by the same method as in Example 1 except that, the gaseous mixture for performing oxacylation reaction was comprised of 41 volume % of propylene, 37 volume % of nitrogen gas, 9 volume % of acetic acid, 6 volume % of oxygen and 7 volume % of water, and the results are listed in Table 1.

Comparative Example 1

The catalyst was prepared by the same method as in Example 1 except that, in step 1), an aqueous $Na_2PdCl_4$ solution with weight of 2.2 kg containing 15 weight % of palladium and an aqueous $HAuCl_4$ solution with weight of 0.5 kg containing 30 weight % of gold were prepared.

This catalyst was evaluated by the same method as in Example 4, and the results are listed in Table 1.

Comparative Example 2

The catalyst was prepared by the same method as in Example 1 except that, in step 1), an aqueous $Na_2PdCl_4$ solution with weight of 2.2 kg containing 15 weight % of palladium and an aqueous $CuCl_2$ solution with weight of 0.5 kg containing 14.6 weight % of copper were prepared.

This catalyst was evaluated by the same method as in Example 4, and the results are listed in Table 1.

Comparative Example 3

The catalyst was prepared by the same method as in Example 1 except that, in step 1), an aqueous $Na_2PdCl_4$ solution with weight of 2.2 kg containing 15 weight % of palladium and an aqueous $HAuCl_4$ solution with weight of 0.5 kg containing 30 weight % of gold were prepared.

This catalyst was evaluated by the same method as in Example 1, and the results are listed in Table 1.

Comparative Example 4

The catalyst was prepared by the same method as in Example 1 except that, in step 1), an aqueous $Na_2PdCl_4$ solution with weight of 2.2 kg containing 15 weight % of palladium and an aqueous $CuCl_2$ solution with weight of 0.5 kg containing 14.6 weight % of copper were prepared

This catalyst was evaluated by the same method as in Example 1, and the results are listed in Table 1.

Comparative Example 5

The catalyst was prepared by the same method as in Example 1 except that, in step 1), an aqueous $Na_2PdCl_4$ solution with weight of 2.2 kg containing 15 weight % of palladium, an aqueous $HAuCl_4$ solution with weight of 0.5 kg containing 30 weight % of gold and an aqueous $CuCl_2$ solution with weight of 0.67 kg containing 15 weight % of copper were prepared.

This catalyst was evaluated by the same method as in Example 1, and the results are listed in Table 1.

Table 1

| Item | STY-1(g/l/hr) | STY-2(g/l/hr) | Relative Ratio | Selectivity(%) |
|---|---|---|---|---|
| Example 1 | 400 | 355 | 0.887 | 94.8 |
| Example 2 | 323 | 204 | 0.632 | 91.6 |
| | | | | |
| Example 3 | 410 | 368 | 0.898 | 95.1 |
| Comparative Example 1 | 400 | 312 | 0.780 | 91.9 |

Table 1   (continued)

| Item | STY-1(g/l/hr) | STY-2(g/l/hr) | Relative Ratio | Selectivity(%) |
|---|---|---|---|---|
| Comparative Example 2 | 420 | 353 | 0.840 | 94.5 |
| Comparative Example 3 | 375 | 122 | 0.325 | 92.8 |
| Comparative Example 4 | 450 | 162 | 0.360 | 95.1 |
| Comparative Example 5 | 385 | 200 | 0.519 | 94.7 |
| Note: 1. STY-1: Space time yield of allyl acetate after the oxacylation reaction was carried out for 6 hours. 2. STY-2: Space time yield of allyl acetate after the oxacylation reaction was carried out for 120 hours. 3. Relative ratio: The catalytic activity deteriorating ratio of STY-2 to STY-1. | | | | |

It is seen clearly from the above Examples and Comparative Examples that, the activity of the traditional catalyst (see Comparative Examples 1 to 5, wherein gold or copper was used as the catalysis promoter) deteriorates rapidly when water is not added during the oxacylation process. As to the catalyst for oxacylation produced in the present invention in which a mixture of tin/gold or tin/copper is used as the promoter, superior catalytic activity deteriorating ratios are obtained when water is not added during the oxacylation process.

Therefore, in the presence of the catalyst produced in this invention, while no water is added into the reactant materials for the oxacylation process, or if only a small amount of water is added in accordance with the requirement of the process, not only can the catalytic activity and life of the catalyst be retained and not deteriorate, but high catalytic activity and high selectivity will be attained. Therefore, the energy consumed and wasted resulting from the addition of water can be avoided and the economical effect of the oxacylation process can be greatly increased.

**Claims**

1.   A catalyst for oxacylation, suitable for producing allyl acetate, which comprises a porous carrier, palladium metal supported on the porous carrier as a main catalyst component, tin metal supported on the porous carrier as a promoter, metal(s) selected from the group consisting of gold, copper, cadmium, bismuth, cerium and a mixture thereof as an additional promoter, and an alkali or alkaline earth metal compound, supported on the porous carrier, wherein the content of said main catalyst, palladium metal, based on weight of said porous carrier, is in the range of 0.1 to 5.0 weight %, the total content of said promoter, tin metal and additional metal(s), based on the weight of said porous carrier, is in the range of 0.01 to 5.0% by weight, and the content of said alkali or alkaline earth metal compound, based on the weight of said porous carrier, is in the range of 4 to 10 weight %.

2.   The catalyst according to claim 1, wherein the content of said main catalyst, palladium metal, based on the weight of said porous carrier, is in the range of 0.3 to 1.5 weight %.

3.   The catalyst according to claim 1, wherein the content of said promoter, tin metal, based on the weight of said porous carrier, is in the range of 0.01 to 5.0 weight.

4.   The catalyst according to claim 3, wherein the content of said promoter, tin metal, based on the weight of said porous carrier, is in the range of 0.02 to 1.0 weight %.

5.   The catalyst according to claim 1, wherein the total content of said promoter, tin metal and additional promoter metal(s), based on the weight of said porous carrier, is in the range of 0.02 to 1.0% by weight.

6.   The catalyst according to claim 1, wherein said additional promoter metal is gold.

7.   The catalyst according to claim 1, wherein said additional promoter metal is copper.

8.   The catalyst according to claim 1, wherein said additional promoter metal is selected from the group consisting of cadmium, bismuth and cerium.

9.   The catalyst according to claim 1, wherein said alkali or alkaline earth metal compounds are the hydroxides, acetates, nitrates and bicarbonates of potassium, sodium, cesium, magnesium and barium.

10. The catalyst according to claim 9, wherein said alkali or alkaline earth metal compounds are the hydroxide, acetate, nitrate and bicarbonate of potassium.

11. The catalyst according to claim 1, wherein said porous carrier is selected from the group consisting of alumina, silica gel, silica, active carbon, silicon carbide, diatomaceous earth, pumice and a mixture thereof.

12. A process for producing allyl acetate which comprises the oxacylation of propylene, acetic acid, oxygen and optionaly water in a vapor phase in the presence of a catalyst according to any preceding claim.

13. A process according to claim 12, wherein the content of water is in the range of 0 to 15 volume %, based on total amount of the reacting gases.

14. A process according to claim 13, wherein the content of water is in the range of 0 to 10 volume %, based on total amount of the reacting gases.

**Patentansprüche**

1. Katalysator zur Oxacylierung, der für die Herstellung von Allylacetat geeignet ist, welcher einen porösen Träger, auf den porösen Träger aufgebrachtes Palladiummetall als Hauptkatalysatorkomponente, auf den porösen Träger aufgebrachtes Zinnmetall als Promotor, Metall(e) als zusätzlichen Promotor, ausgewählt aus der Gruppe, die aus Gold, Kupfer, Cadmium, Bismut, Cer und deren Gemischen besteht, und eine auf den porösen Träger aufgebrachte Alkali- oder Erdalkalimetallverbindung umfasst, wobei der Gehalt des Hauptkatalysators Palladiummetall, bezogen auf den porösen Träger, im Bereich von 0,1 bis 5,0 Gew.-% liegt, der Gesamtgehalt des Promotors Zinnmetall und des zusätzlichen Metalls oder der zusätzlichen Metalle, bezogen auf das Gewicht des porösen Trägers, im Bereich von 0,01 bis 5,0 Gew.-% liegt, und der Gehalt der Alkali- oder Erdalkalimetallverbindung, bezogen auf das Gewicht des porösen Trägers, im Bereich von 4 bis 10 Gew.-% liegt.

2. Katalysator nach Anspruch 1, worin der Gehalt des Hauptkatalysators Palladiummetall, bezogen auf das Gewicht des porösen Trägers, im Bereich von 0,3 bis 1,5 Gew.-% liegt.

3. Katalysator nach Anspruch 1, worin der Gehalt des Promotors Zinnmetall, bezogen auf das Gewicht des porösen Trägers, im Bereich von 0,01 bis 5,0 Gew.-% liegt.

4. Katalysator nach Anspruch 3, worin der Gehalt des Promotors, Zinnmetall, bezogen auf das Gewicht des porösen Trägers, im Bereich von 0,02 bis 1,0 Gew.-% liegt.

5. Katalysator nach Anspruch 1, worin der Gesamtgehalt des Promotors Zinnmetall und des zusätzlichen Promotermetalls bzw. der zusätzlichen Promotormetalle, bezogen auf das Gewicht des porösen Trägers, im Bereich von 0,02 bis 1,0 Gew.-% liegt.

6. Katalysator nach Anspruch 1, worin das zusätzliche Promotormetall Gold ist.

7. Katalysator nach Anspruch 1, worin das zusätzliche Promotormetall Kupfer ist.

8. Katalysator nach Anspruch 1, worin das zusätzliche Promotormetall ausgewählt ist aus der Gruppe, die aus Cadmium, Bismut und Cer besteht.

9. Katalysator nach Anspruch 1, worin die Alkali- und Erdalimetallverbindungen die Hydroxide, Acetate, Nitrate und Hydrogencarbonate von Kalium, Natrium, Cäsium, Magnesium und Barium sind.

10. Katalysator nach Anspruch 9, worin die Alkali- und Erdalkalimetallverbindungen das Hydroxid, das Acetat, das Nitrat und das Hydrogencarbonat des Kaliums sind.

11. Katalysator nach Anspruch 1, worin der poröse Träger aus der Gruppe ausgewählt ist, die aus Aluminiumoxid, Kieselgel, Siliciumdioxid, Aktivkohle, Siliciumcarbid, Diatomeenerde, Bimsstein und deren Gemischen besteht.

12. Verfahren zur Herstellung von Allylacetat, das die Oxacylierung von Propylen, Essigsäure, Sauerstoff und gegebenenfalls Wasser in der Dampfphase in Gegenwart eines Katalysators nach einem der vorhergehenden Ansprüche umfasst.

13. Verfahren nach Anspruch 12, worin der Wassergehalt im Bereich von 0 bis 15 Volumen-%, bezogen auf die Gesamtmenge der Reaktionsgase, liegt.

14. Verfahren nach Anspruch 13, worin der Wassergehalt im Bereich von 0 bis 10 Volumen-%, bezogen auf die Gesamtmenge der Reaktionsgase, liegt.

## Revendications

1. Catalyseur pour l'oxacylation, approprié pour produire de l'acétate d'allyle, qui comprend un support poreux, du palladium métallique supporté sur le support poreux en tant que composant catalytique principal, de l'étain métallique supporté sur le support poreux en tant que promoteur, un ou des métaux choisis dans le groupe constitué par l'or, le cuivre, le cadmium, le bismuth, le cérium et un mélange de ceux-ci en tant que promoteur supplémentaire, et un composé de métal alcalin ou alcalino-terreux, supporté sur le support poreux, dans lequel la teneur dudit catalyseur principal, à savoir du palladium métallique, sur la base du poids dudit support poreux, se situe dans la plage de 0,1 à 5,0% en poids, la teneur totale dudit promoteur, à savoir de l'étain métallique et du ou des métaux supplémentaires, sur la base du poids dudit support poreux, se situe dans la plage de 0,01 à 5,0% en poids, et la teneur dudit composé de métal alcalin ou alcalino-terreux, sur la base du poids dudit support poreux, se situe dans la plage de 4 à 10% en poids.

2. Catalyseur selon la revendication 1, dans lequel la teneur dudit catalyseur principal, à savoir du palladium métallique, sur la base du poids dudit support poreux, se situe dans la plage de 0,3 à 1,5% en poids.

3. Catalyseur selon la revendication 1, dans lequel la teneur dudit promoteur, à savoir de l'étain métallique, sur la base du poids dudit support poreux, se situe dans la plage de 0,01 à 5,0% en poids.

4. Catalyseur selon la revendication 3, dans lequel la teneur dudit promoteur, à savoir de l'étain métallique, sur la base du poids dudit support poreux, se situe dans la plage de 0,02 à 1,0% en poids.

5. Catalyseur selon la revendication 1, dans lequel la teneur totale dudit promoteur, à savoir de l'étain métallique et du ou des métaux promoteurs supplémentaires, sur la base du poids dudit support poreux, se situe dans la plage de 0,02 à 1,0% en poids.

6. Catalyseur selon la revendication 1, dans lequel ledit métal promoteur supplémentaire est l'or.

7. Catalyseur selon la revendication 1, dans lequel ledit métal promoteur supplémentaire est le cuivre.

8. Catalyseur selon la revendication 1, dans lequel ledit métal promoteur supplémentaire est choisi dans le groupe constitué par le cadmium, le bismuth et le cérium.

9. Catalyseur selon la revendication 1, dans lequel lesdits composés de métaux alcalins ou alcalino-terreux sont les hydroxydes, acétates, nitrates et bicarbonates de potassium, sodium, césium, magnésium et baryum.

10. Catalyseur selon la revendication 9, dans lequel lesdits composés de métaux alcalins ou alcalino-terreux sont l'hydroxyde, l'acétate, le nitrate et le bicarbonate de potassium.

11. Catalyseur selon la revendication 1, dans lequel ledit support poreux est choisi dans le groupe constitué par l'alumine, le gel de silice, la silice, le charbon actif, le carbure de silicium, la terre à diatomées, la pierre ponce et un mélange de ceux-ci.

12. Procédé de fabrication d'acétate d'allyle qui comprend l'oxacylation du propylène, de l'acide acétique, de l'oxygène et facultativement de l'eau, dans une phase vapeur, en présence d'un catalyseur tel que défini à l'une quelconque des revendications précédentes.

13. Procédé selon la revendication 12, dans lequel la teneur en eau se situe dans la plage de 0 à 15% en volume, sur la base de la quantité totale des gaz réactifs.

14. Procédé selon la revendication 13, dans lequel la teneur en eau se situe dans la plage de 0 à 10% en volume, sur la base de la quantité totale des gaz réactifs.